# DEMANDE DE BREVET EUROPEEN

(11) **EP 3 236 450 A1**
(43) Date de publication de la demande: **25.10.2017**
(21) Numéro de dépôt: 17166878.3
(22) Date de dépôt: 18.04.2017
(51) Int. Cl.: G09B 19/00, A61B 5/00, A61B 5/11, H04M 1/04, F16M 13/04, A63B 24/00

(54) **DISPOSITIF DE REEDUCATION FONCTIONNELLE OU D ENTRAINEMENT SPORTIF, INTERACTIF, AUTONOME ET PORTABLE**

(30) Priorité: 22.04.2016 FR 1600672
(71) Demandeur: Aubert, Bruno, 66650 Banyuls sur mer (FR)
(72) Inventeur: Aubert, Bruno, 66650 Banyuls sur mer (FR); Castang, Jean-Baptiste, Newnan, GA 30265 (US); Bonin, Eric, 69930 Saint Clément les Places (FR)

(57) **Abrégé**

Le procédé consiste à fixer directement ou indirectement sur le membre que l'on souhaite rééduquer ou entraîner d'un patient, un détecteur/enregistreur de mouvements afin d'en suivre et enregistrer en continu les dits mouvements réalisés, pour les comparer en temps réel ou en différé à ceux préconisés suivant une consigne préétablie par un kinésithérapeute ou un entraineur sportif, et d'avertir ces derniers ou le dit patient, par un signal sonore, parlé ou vidéo, d'une quelconque dérive afin que le patient corrige ses mouvements.

La fixation du détecteur/enregistreur de mouvements, idéalement un smartphone doté d'accéléromètres, se fait par l'intermédiaire d'un support dont la structure rigide permet de transmettre les mouvements sans perte d'énergie et qui est doté d'éléments permettant un positionnement précis et répétable du membre par rapport au smartphone. Le dispositif comprend donc un support autonome et portable, permettant d'y fixer un smartphone et d'y positionner un membre (main, pied, jambe, bras), et un logiciel/application pour analyser les mouvements réalisés en les comparants à une consigne préalablement paramétrée, grâce à une interface conviviale et interactive, par un kinésithérapeute ou un entraineur sportif voire le patient.

## Description

La présente invention concerne la rééducation rigoureuse d'un membre d'une personne blessée, ou l'entraînement de sportifs tant en institut chez les professionnels que chez le patient, pour assurer une parfaite guérison ou un optimum entrainement.

La rééducation d'un membre d'une personne blessée, ou l'entraînement de sportifs, doivent souvent être réalisés sous la surveillance de professionnels afin d'éviter les mauvais gestes et surtout d'assurer un programme précis et suivi.

Malheureusement, ces professionnels sont souvent débordés et ne peuvent assurer convenablement ces rééducations ou entraînements. De surcroit, après la séance d'entrainement ou de rééducation, le patient est livré à lui-même et ne peut poursuivre les mouvements préconisés sans courir le risque de faire des erreurs entrainant parfois une blessure.

La présente invention se propose de résoudre ce problème de façon simple, pour le professionnel voire pour le patient lui-même, avec un procédé et son dispositif associé, nouveaux et innovants qui permet de surveiller en permanence les mouvements réalisés, de les comparer à une consigne et d'avertir le kinésithérapeute ou l'entraineur sportif d'un éventuel écart pour y remédier rapidement.

Les professionnels de la santé ou du sport pourront ainsi s'occuper de plus de personnes à la fois et pourront même proposer des exercices à domicile pour prolonger la rééducation ou augmenter l'entrainement sportif tout en surveillant les gestes réalisés à distance.

### Art antérieur

Il existe peu d'appareils permettant une rééducation fonctionnelle ou un entraînement sportif, susceptibles d'être réalisés de façon simple, par le patient lui-même avec bien sûr une surveillance directe ou indirecte d'un professionnel tel qu'un kinésithérapeute ou un entraineur sportif.

Il y a bien le dispositif KinEvolution objet du brevet WO2010067034 qui décrit un appareil d'exercice musculaire et/ou articulaire adapté à la rééducation et/ou l'entraînement physique d'une partie du corps d'un utilisateur mais cet appareil est d'une grande complexité puisqu'il comprend de multiples composants de grandes tailles (bras d'articulation, vérins, freins, axes, corps coulissant, bâti...), probablement très chers même pour un professionnel. Ce dispositif, s'il est interactif, n'est ni portable, ni autonome et ne permet pas d'être utilisé par le patient lui-même à la maison.

Il existe aussi le dispositif objet du brevet WO 2015130177 A1 qui revendique une planche d'équilibre qui comprend une ouverture/cavité pour recevoir un dispositif électronique portable ayant un capteur pour détecter les mouvements tel un téléphone portable, mais à aucun moment il n'y est fait mention ou revendiqué un positionnement précis et répétable des pieds par rapport au téléphone portable.

En fait, cette planche d'équilibre est plutôt faite pour faire des jeux qui n'ont pas pour objectif la rééducation ou l'entraînement de membres suivant un protocole bien défini par un kinésithérapeute ou un entraineur sportif.

Enfin, le brevet WO2015041618 quant à lui, décrit un robot de rééducation qui impose mécaniquement un mouvement au membre à rééduquer ce qui lui confère une grande complexité et son imposante taille induit très probablement un prix élevé, ne le prédestinant pas à un usage par tous les professionnels et surtout le patient lui-même. Enfin, on voit apparaître des dispositifs se fixant à un membre comme le **Arm Skate** mais aucun pilotage interactif de la rééducation n'y est décrite ni revendiquée.

### Descriptif de l'invention

Le procédé consiste à fixer directement ou indirectement sur le membre que l'on souhaite rééduquer ou entraîner d'un patient, un détecteur/enregistreur de mouvements afin d'en suivre et enregistrer en continu les dits mouvements réalisés, pour les comparer en temps réel ou en différé à ceux préconisés suivant une consigne préétablie par un kinésithérapeute ou un entraineur sportif, et d'avertir ces derniers ou le dit patient, par un signal sonore, parlé ou vidéo (y compris les interfaces de jeux vidéo), d'une quelconque dérive afin que le patient corrige ses mouvements.

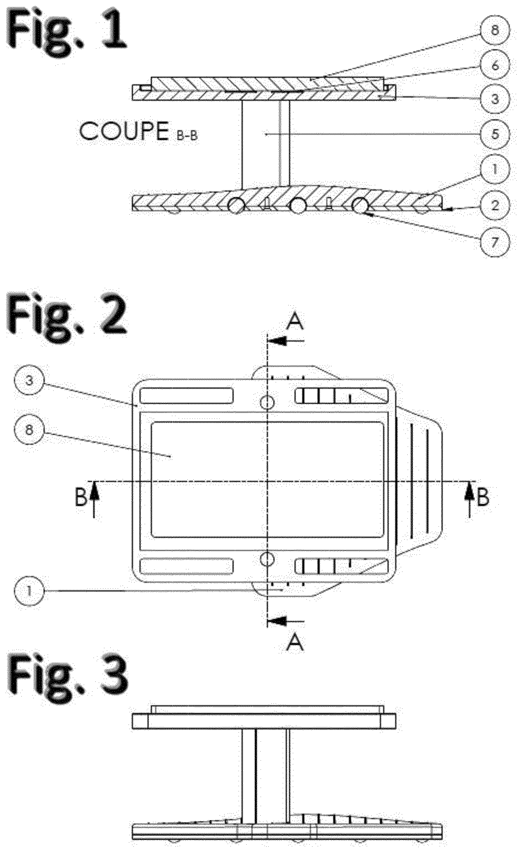

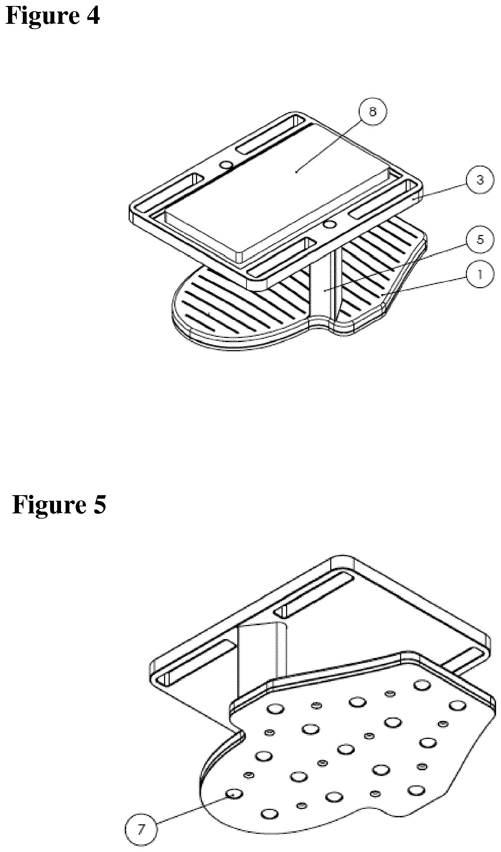

La fixation du détecteur/enregistreur de mouvements, idéalement un smartphone doté au moins d'accéléromètres linéaires x, y et z, se fait préférentiellement par l'intermédiaire d'un support dont la structure rigide permet de transmettre les mouvements sans perte d'énergie et qui est doté d'éléments permettant un positionnement précis et répétable du membre par rapport au smartphone. Un exemple de dispositif pour être utilisé avec la main, présenté dans les figures 1 à 5, comprend:
* un support rigide (1) autonome et portable, permettant d'y fixer un détecteur/enregistreur de mouvements comme un smartphone (8) et d'y positionner précisément et de façon répétable, par rapport au dit détecteur/enregistreur de mouvements, grâce à des butées (5), une main (généralisable aux pied, jambe, bras),
* un logiciel/application pour analyser les mouvements réalisés en les comparants à une consigne préalablement paramétrée, grâce à une interface conviviale et interactive, par un kinésithérapeute ou un entraineur sportif voire le patient.

Le support présente une structure rigide pour permettre de transmettre sans perte d'énergie les accélérations du membre concerné directement au détecteur/enregistreur de mouvements.

Suivant l'une des dispositions préférées, le support présente une surface plane (2) pour permettre de réaliser les mouvements de rééducation sur une table, un meuble, un mur ou une vitre.

Suivant une autre des dispositions préférées, la surface plane du support est dotée de billes (7) sur ressorts (non représentés) afin de permettre au patient d'exercer un effort, avec déplacement, suivant l'axe orthogonal à la dite surface.

La surface plane du support peut également être réalisée en matériau déformable, de façon réversible, afin de permettre au patient d'exercer un effort, avec déplacement, suivant l'axe orthogonal à la dite surface.

Ce smartphone est donc doté d'un logiciel interactif qui est programmé par le kinésithérapeute pour permettre de suivre et d'enregistrer en continu le mouvement qu'il a préconisé pour le patient qui doit le reproduire.

Le mouvement est suivi en x,y et z grâce aux accéléromètres du smartphone.

Par exemple pour un mouvement (préconisé par un kinésithérapeute) tel que la forme d'un rond de 30 cm réalisé sur une table, les accéléromètres x et y seuls seront sollicités et leurs courbes obtenues représenteront deux sinusoïdales identiques en amplitude mais déphasées de Pi. La moindre différence d'amplitude ou de déphasage renseignera sur le fait que le geste est mal réalisé et que le patient doit le corriger.

Dans le cas d'un suivi nécessitant la fixation du smartphone non pas sur une extrémité (main, pied) mais sur par exemple un bras, le smartphone sera fixé directement sur le dit bras grâce à un simple strap. Ce peut être le cas lorsque le professionnel de la santé ou du sport souhaite surveiller les contractions d'un muscle blessé. Dans ce cas, il n'y a pas de support et le logiciel tiendra compte de ces conditions d'amortissement dû à l'élasticité du muscle.

Les figure 1 à 5 montrent le dispositif destiné par exemple à la rééducation de la coiffe du rotateur de l'épaule, suivant l'une des dispositions préférées.

Le support (1) muni d'une forme pour la paume de la main (ou la plante du pied) et de butées (5), adaptées à la main (gauche ou droite) et d'un dispositif (6) de fixation (de type Velcro, aimant...) du smartphone (8) sur le support (3).

Le logiciel/application spécifique permet :
* la saisie des données par le kinésithérapeute ou le client, concernant les mouvements de rééducation fonctionnelle ou d'entraînement à réaliser (par exemple un rond de diamètre 30 cm réalisé 30 fois par minute),
* de suivre et enregistrer les dits mouvements du membre et les comparer à ceux préconisés,
* d'avertir par un signal sonore, parlé ou vidéo, le patient d'une quelconque dérive afin qu'il puisse corriger son mouvement,
* de transmettre les données et autres informations par USB, WiFi, Bluetooth, Internet..., vers un ordinateur central ou sur un support (téléphone, ordinateur...) du kinésithérapeute, de l'entraineur sportif ou du patient.

## Revendications

1. Dispositif de rééducation fonctionnelle ou d'entrainement, **caractérisé en ce qu'**il comprend un détecteur/enregistreur de mouvements destiné à être fixé directement ou indirectement sur le membre concerné d'un patient afin d'en suivre et enregistrer en continu les dits mouvements réalisés pour les comparer à ceux préconisés suivant une consigne préétablie et avertir le dit patient par un signal sonore, parlé ou vidéo, d'une quelconque dérive afin qu'il corrige ses mouvements.

2. Dispositif de rééducation fonctionnelle ou d'entrainement suivant la revendication 1, **caractérisé en ce que** le détecteur/enregistreur de mouvement est un smartphone doté au moins d'accéléromètres linéaires x, y et z.

3. Dispositif de rééducation fonctionnelle ou d'entrainement suivant l'une quelconque des revendications précédentes, **caractérisé en ce que** le détecteur/enregistreur de mouvements comprend un support par l'intermédiaire duquel il est fixé au membre concerné et permettant de positionner précisément et de façon répétable le dit membre par rapport au détecteur/enregistreur de mouvements.

4. Dispositif de rééducation fonctionnelle ou d'entrainement suivant la revendication 3, **caractérisé en ce que** le support présente une structure rigide pour permettre de transmettre sans perte d'énergie les accélérations du membre concerné directement au détecteur/enregistreur de mouvements.

5. Dispositif de rééducation fonctionnelle ou d'entrainement suivant la revendication 3, **caractérisé en ce que** le support présente une surface plane pour permettre de réaliser les mouvements de rééducation sur une table, un meuble, un mur ou une vitre.

6. Dispositif de rééducation fonctionnelle ou d'entrainement suivant la revendication 5, **caractérisé en ce que** la surface plane du support est dotée de billes sur ressorts afin de permettre au patient d'exercer un effort, avec déplacement, suivant l'axe orthogonal à la dite surface.

7. Dispositif de rééducation fonctionnelle ou d'entrainement suivant la revendication 5, **caractérisé en ce que** la surface plane du support est réalisée en matériau déformable de façon réversible afin de permettre au patient d'exercer un effort, avec déplacement, suivant l'axe orthogonal à la dite surface.

8. Dispositif de rééducation fonctionnelle ou d'entrainement suivant l'une quelconque des revendications 3 à 7 et la revendication 2, **caractérisé en ce que** le:
* support (1) est muni d'une forme et de butées (5) adaptées au positionnement précis du membre à rééduquer ou entrainer et **en ce qu'**il comporte un dispositif (6) de fixation du smartphone (8) sur le support (3),
* le logiciel/application spécifique dans le smartphone permet :
+ la saisie de données par le kinésithérapeute ou le client, concernant les mouvements de rééducation fonctionnelle ou d'entraînement à réaliser,
+ de suivre et enregistrer les mouvements du membre concerné,
+ d'avertir par un signal sonore, parlé ou vidéo, le patient d'une quelconque dérive afin qu'il puisse corriger son mouvement,
+ de transmettre les données et autres informations par USB, WiFi, Bluetooth, Internet.
